(19) Europäisches Patentamt
European Patent Office
Office européen des brevets

(11) Publication number: **0 101 991**
**B1**

(12) **EUROPEAN PATENT SPECIFICATION**

(45) Date of publication of patent specification: **15.01.86**

(51) Int. Cl.$^4$: **C 07 F 9/40**

(21) Application number: **83107817.5**

(22) Date of filing: **25.11.80**

(60) Publication number of the earlier application in accordance with Art. 76 EPC: **0 031 041**

(54) **2-Alkene-phosphonates and process for their production.**

(30) Priority: **26.11.79 IT 2754279**
**05.06.80 IT 2256680**

(43) Date of publication of application:
**07.03.84 Bulletin 84/10**

(45) Publication of the grant of the patent:
**15.01.86 Bulletin 86/03**

(84) Designated Contracting States:
**BE CH DE FR GB LI NL**

(56) References cited:

**CHEMICAL ABSTRACTS, vol. 69, no. 10, 2nd September 1968, page 4044, no. 43254c, Columbus, Ohio, US, G. MAVEL et al.: "N.M.R. study of organophosphorus compounds. XIV. Conformation of substituted allylphosphonates"**
**CHEMICAL ABSTRACTS, vol. 59, no. 1, 8th July 1963, no. 656d, Columbus, Ohio, US, B.I. IONIN et al.: "Prototropic isomerization of esters of alkenylphosphonic acids"**

(73) Proprietor: **Montedison S.p.A.**
**31, Foro Buonaparte**
**I-20121 Milan (IT)**

(72) Inventor: **Bosone, Enrico, Dr.**
**1, Via Modestino**
**Milano (IT)**
Inventor: **Caprara, Giuseppe**
**17/1, Via Tolentino**
**Milano (IT)**
Inventor: **Corda, Francesco**
**13, Via Teodosio**
**Milano (IT)**
Inventor: **Gozzo, Franco, Dr.**
**52, Via Triulziana**
**San Donato Milanese (IT)**
Inventor: **Menconi, Augusto**
**Via Lago Gerundo Pal. Coesmi B**
**I-26013 Crema (Cremona) (IT)**
Inventor: **Piccardi, Paolo**
**8, Via E. De Marchi**
**Milano (IT)**
Inventor: **Carprioli, Vincenzo**
**8, Via Loriga**
**San Martino (Pavia) (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

**Description**

The present invention concerns intermediates for the production of new insecticides and acaricides belonging to the class of pyrethriods.

These new compounds have the general formula I:

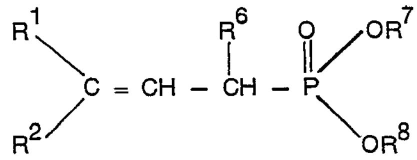

(I)

wherein:

$R^1$ = F, Cl, Br, $CH_3$, $CF_3$;

$R^2$ = F, Cl, Br, $CF_3$;

$R^6$ = H, F, Cl, Br;

$R^7$ and $R^8$, equal to or different from each other, = alkyl or haloalkyl with $C_1$—$C_4$, or $R^7$ and $R^8$ together constitute a

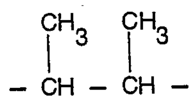 group or a 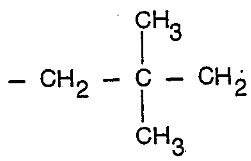 group,

provided that when $R^1$ = $CH_3$, $R^2$ will be different from Cl.

The new compounds may be prepared by the following reactions 1 to 8

1) 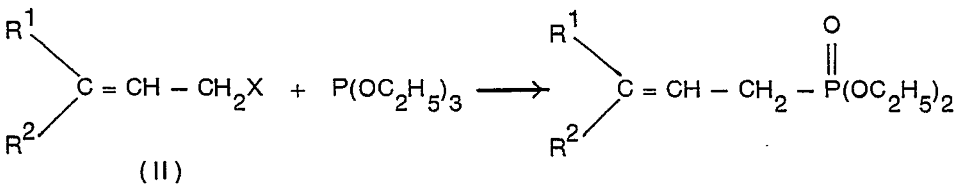

(II)

[I, $R^6$ = H, $R^7 = R^8 = C_2H_5$]

2) 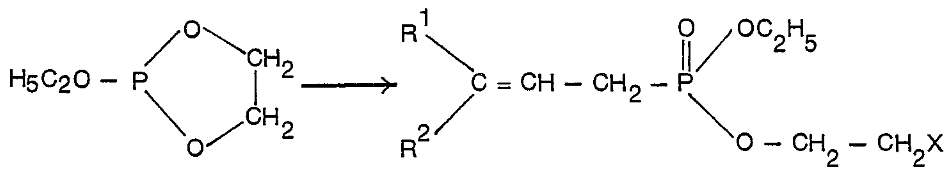

[I, $R^7$ (or $R^8$) = haloalkyl]

3) 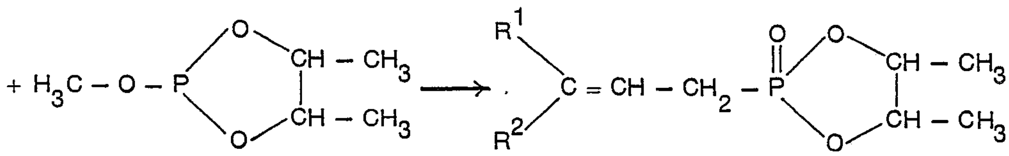

[I, $R^7$ & $R^8$ together = 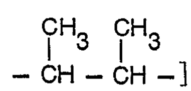]

4) 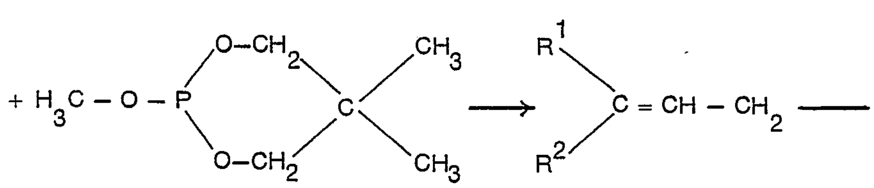

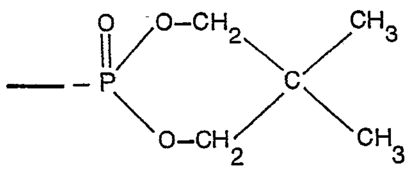

[I, $R^7$ & $R^8$ together = 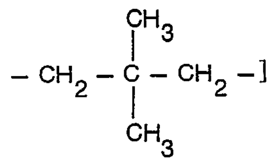 ]

5) [I, $R^6$ = H] 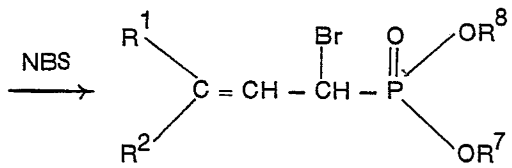


[I, $R^6$ = Br]

6) [I, $R^6$ = H] 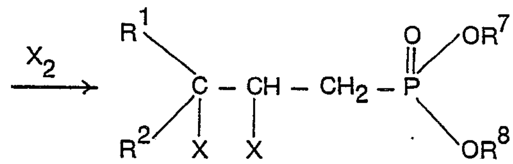

(III)

7) (III) 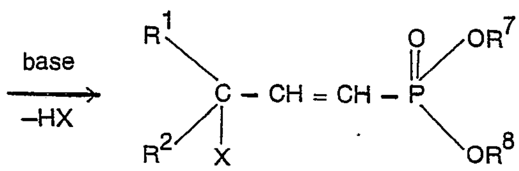

(IV)

8) (IV) 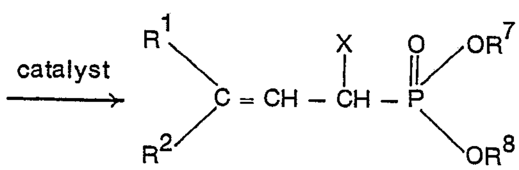

[I, X = halogen]

In the above reported reaction $R^1$, $R^2$, $R^6$, $R^7$ and $R^8$, if not otherwise indicated, have the meanings reported for formula I' X stands for chlorine or bromine while NBS indicates N-bromo-succinimide. Reactions from 1 to 4 represent examples of methods for the preparation of the compounds of formula I wherein $R^6$=H. The compounds wherein $R^6$ = halogen, may be prepared in a similar way starting from suitable 2-alkenyl halides, or they may be prepared from the compounds of formula I wherein $R^6$ = H, by the methods illustrated by reactions from 5 to 8.

All starting compounds of reaction 1 to 4 are known compounds or they can be easily prepared according to known methods. Reaction 5 concerns a standard halogenation in allylic position, carried out with N-bromo-succinimide.

Reactions 6 to 8 represent an alternative to reaction 5, also useful for preparing compounds in which $R^6$ = Cl. The addition of halogen ($X_2$) to compounds I wherein $R^6$=H (reaction 6) is carried out according to techniques that are standard in organic chemistry, using halogens or halogenating agents such as $SO_2Cl_2$.

The dehydrohalogenation of adduct (III) (reaction 7) may be carried out either in the presence of inorganic bases or of organic bases such as secondary or tertiary amines.

The reaction of the re-arrangement of adducts (IV) (reaction 8) is a new one and as such forms a further object of this invention.

Said reaction accours quantitatively at a temperature comprised between 20° and 150°C, in the presence of catalytic quantities of a bivalent copper halide, by simple stirring of the mixture consisting of the adduct (IV) and of cupric halide.

In case X = Cl, a suitable catalyst is cupric chloride in the commonly used form of dihydrate.

The new intermediates are useful in the preparation of valuable insecticides and acaricides of the pyrethroid series.

The new insecticides have the general formula (A)

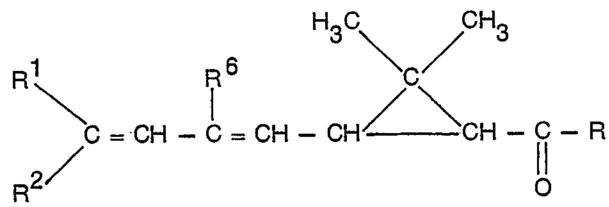

(A)

wherein

$R^1$ = F, Cl, Br, $CH_3$, $CF_3$,
$R^2$ = F, Cl, Br, $CF_3$,
$R^6$ = H, F, Cl, Br,

R =

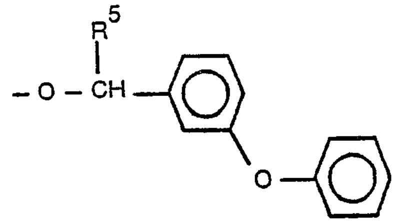

in which $R^5$ = H, CN, —C≡CH.

The new insecticides (A) were prepared starting from the new intermediates by reacting in the presence of a strong base a 2-alkene-phosphonate of the above mentioned formula (I)

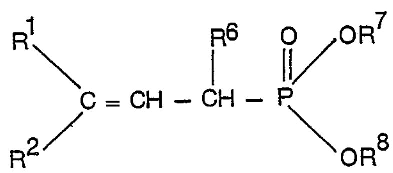

(I)

wherein the residues $R^1$, $R^3$, $R^6$, $R^7$ and $R^8$ are defined as above, with a lower alkylester of caronic aldehyde (V) (2,2-dimethyl-3-formyl-cyclopropanecarboxylic acid) of the formula:

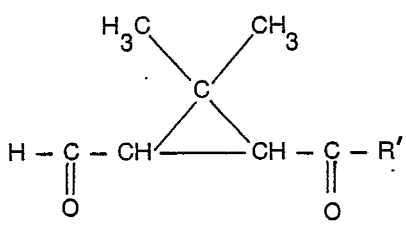

(V)

(wherein: Ŕ = O-alkyl $C_1$—$C_4$).

The above process, is illustrated by reaction 9):

9) 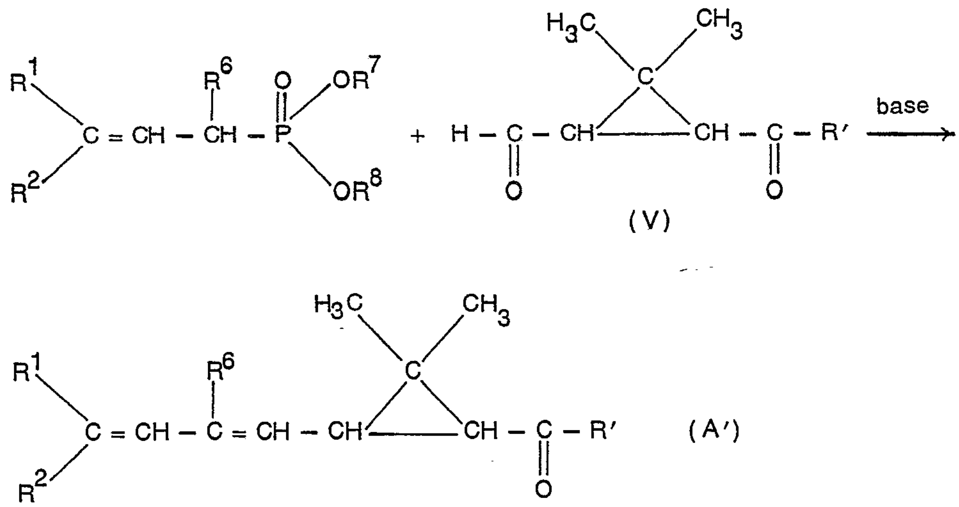


(V)

(A')

The compounds of formula I have been indicated as phosphonates, thereby using this term in its more general meaning, that is, referred to organic compounds of the pentavalent phosphorus in which there is present a carbon-phosphorus bond.

It must, however, be pointed out that the correct nomenclature for the compounds of formula I (in which substituents $R^7$ and $R^8$ together represent either a

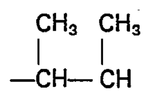

or a

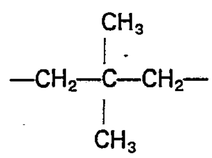

group and thus form with the

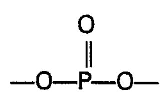

group either a pentaatomic or hexaatomic ring) foresees for these compounds the term 2-oxo-1,3,2-dioxaphospholane and respectively 2-oxo-1,3,2-dioxa-phosphorinane.

In this context, the compounds of formula I will in the following be indicated by the term "phosphonates", also comprising thereby the compounds of formula I in which there occurs either a penta- or hexatomic ring.

As far as we know, only one example of a reaction between a 2-alken-phosphonate and an aldehyde was known from the literature. Said example concerns the reaction between methyl 3-chloro-2-butene-phosphonate and benzaldehyde [G. Lavielle, C. R. Acad. Sci., Ser. C, 86 (1970)].

The above cited reaction leads to the formation of three products, only one of them having a dienic structure:

10) 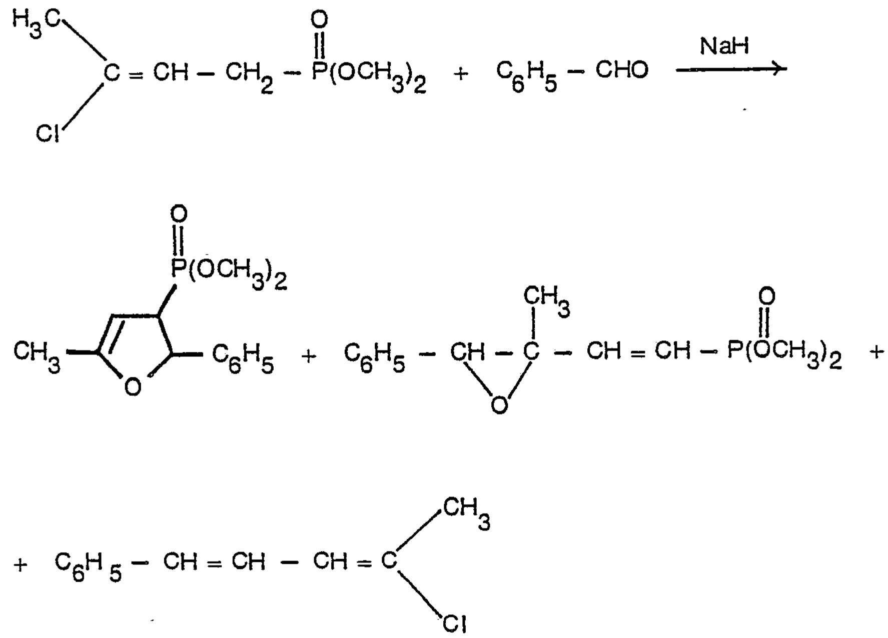


It had thus to be expected that also from reaction 9 there would form compounds of little interest for the purposes of the desired structure.

On the contrary, we surprisingly found that reaction 9 affords compounds of formula A′ with high yields and allows to separate (isolate) the desired product quite easily and with a high degree of purity.

Reaction 9 is carried out using substantially equimolar quantities of phosphonate I and of the ester of caronic aldehyde (V), in the presence of substantially equimolecular amounts of a strong base, in an inert solvent or diluent and at temperatures comprised between −50°C and +100°C, but preferably comprised between −30°C and room temperature. Suitable strong bases may be hydrides or hydroxides of alkaline metals such as sodium hydride, lithium hydride, sodium hydroxide, potassium hydroxide, etc.

In case that as a base there is used sodium hydride, it is preferable to operate in the presence of catalytic amounts of suitable activators such as for instance ethyl alcohol or imidazole. As a reaction medium there may be used an ether such as for instance: dimethoxy-ethane or tetrahydrofurane, an aprotic polar solvent such as N,N-dimethylformamide or a pair of protic-aprotic solvents such as e.g. water and an aromatic hydrocarbon (in this latter case, as a base there must be used an alkaline hydroxide).

In a practical embodiment, the reaction 9 may be conducted by preparing a mixture of phosphonate I and of the ester of caronic aldehyde (V), in a molar ratio of about 1:1 and by then admixing slowly this mixture to a solution or a suspension of the base in a suitable inert solvent.

In another practical form of embodiment, reaction 9 is conducted by slowly admixing a solution of phosphonate I in an inert solvent to a mixture consisting of the base, of the ester of caronic aldehyde (V) and of an inert solvent. Reaction 9, in general, is carried out in an anhydrous medium and under an atmosphere of an inert gas when a base there is used an alkaline metal-hydride. The lower alkyl esters of caronic aldehyde (V) are known compounds that are separable in two isomeric forms, cis and trans (isomerism on the cyclopropane ring).

The possibility to use in reaction 9 also only just one of the two cis or trans forms, allows to obtain cyclopropanecarboxylic esters (A′) having the corresponding isomerism.

As far as is known to us, the only 2-alkene-phosphonate of formula I known previously, is methyl 3-chloro-2-butene-phosphonate (reaction 10). The thus obtained esters (A′) are then converted into the corresponding acyl halides, and the obtained acyl halides are reacted with alcohols of the general formula (VI)

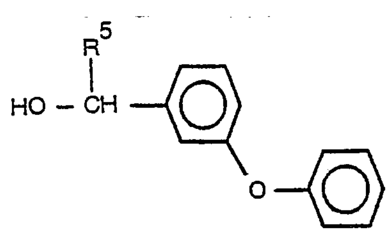

(VI)

wherein

$R^5$ has the above meaning.

The preparation is carried out by methods known in the art, e.g. by saponification of the ester into the

free acid and reacting the acid with thionyl chloride. Also the esterification of the acid halides with the alcohols (VI) is carried out by usual methods.

The compounds of general formula A, in general, are obtained as mixtures of geometrical and configurational isomers due to the particular structure of the molecule which contains asymmetric carbon atoms and double bonds.

The separation of the various mixtures in the various diastereoisomers may be achieved following the techniques usually applied in the normal practice of organic chemistry such as for instance chromatographic methods.

In order to even further illustrate this invention, in the following are given a number of examples.

Example 1

Preparation of the diethylester of 3,3-dichloro-2-propenephosphonic acid (reaction 1):

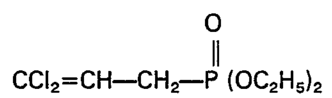

Into a 250 ml flask, fitted with a magnetic stirrer and a reflux condenser, there were introduced:
83 g (0.5 mols) of triethylphosphite [$P(OC_2H_5)_3$]
73 g (0.5 mols) of 1,1,3-trichloropropene $Cl_2C=CH—CH_2Cl$.

The reaction mixture was stirred for 5 hours at 160°C.

It was then allowed to cool down to room temperature, whereafter it was distilled at reduced pressure thereby gathering the fraction with boiling point comprised between 145° and 153°C at a pressure of 24 mbar (104.7 g).

The fraction thus gathered proved to consist of the product desired, having a purity of 96.6% (gas chromatographic titre GLC).

*IR*(infrared spectroscopy), ($cm^{-1}$): 1620 (v C=C); 1260 (v P=O); 1025, 965, 920.

$^1H$ *NMR* (nuclear magnetic resonance): δ, ppm ($CDCl_3$, TMS)

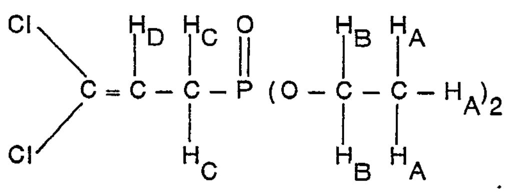

1.1—1.5 (t, 6H, $CH_3$)
2.4—3.0 (dd, 2H, $CH_2$—P)
3.8—4.4 (m, 4H, $OCH_2$)
5.7—6.2 (m, 1H, $CCl_2=CH$)
$^JH_A—H_B$ = 7 Hz
$^JH_C—P$ = 22 Hz
$^JH_C—H_D$ = 8 Hz

(t = triplet; dd = doublet of doublet, m = multiplet; J = coupling constant).

Example 2

Preparation of the diethyl ester of 3,3-dichloro-1-bromo-2-propene-phosphonic acid (reaction 2):

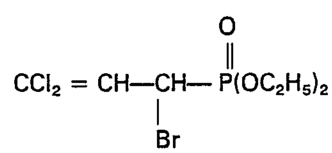

Into a 1 litre flask, fitted with a mechanical stirrer and with a reflux condenser, closed by a calcium chloride valve, there were introduced:
125 g (0.5 mols) of diethylester of 3,3-dichloro-2-propenephosphonic acid (obtained as described in example 28).
100 g (0.56 mols) of N-bromosuccinimide,
500 ml of carbon tetrachloride,
1.0 g of azo-bis-isobutyronitrile (A.B.N.).

This mixture was then refluxed heated, under constant stirring for 2 hours, after which there were admixed further 1.0 g of A.B.N. and refluxing was carried on under stirring for another 2 hours. After cooling down to 0°C, the mixture was then filtered in order to remove the succinimide and from the filtrate was then evaporated the solvent.

The residue was diluted in 500 ml of petroleum ether and then was cooled down to 0°C. Once again there precipitated a small quantity of succinimide which was removed by filtering.

From this filtrate, after evaporation of the solvent, there was obtained a residue which was purified by chromatography on a silica gel column (eluent: petroleum ether-$CH_2Cl_2$-ethyl acetate in the ratio of 10:6:4).

Thereby were obtained 135 g of the desired product.

The product analysis showed:

*IR*: in agreement with the assigned structure;

*Elemental analysis*:
C, in %: theoretical: 25.75 — found: 25.43
H, in %: theoretical: 3.71 — found: 3.64
Cl, in %: theoretical: 21.75 — found: 21.77
Br,in % theoretical: 24.52 — found 25.39
P, in %: theoretical: 9.50 — found: 9.22

*NMR*δ, ppm ($CDCl_3$, TMS

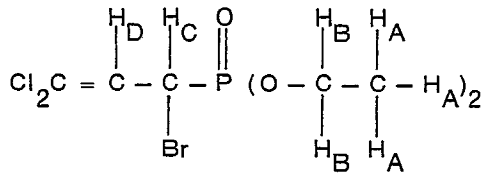

1.35 (t, 6H, $CH_3$)
4.22 (dq, 4H, O—$CH_2$)
4.68 (dd, 1H, CHBr)
6.16 (dd, 1H, =CH)
$^JH_A$—$H_B$ =7 Hz
$^JH_B$—P = 8.5 Hz
$^JH_C$—$H_D$ = 11.5 Hz
$^JH_C$—P = 11.5 Hz
$^JH_D$—P = 8 Hz.
(t = triplet; dq = doublet of quartet; dd = doublet of doublet; J = coupling constant).

Example 3

Preparation of the diethyl ester of 2,3,3,3-tetrachloropropane-phosphonic acid (Reaction 6)

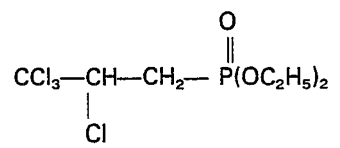

Into a 500 ml flask, fitted with a mechanical stirrer, a thermometer and a reflux condenser connected with a $H_2SO_4$ valve and with one column for the separation of the gases, there were introduced:
150 ml of benzene,
24.7 g of diethyl ester of 3,3-dichloro-2-propene-phosphonic acid (prepared as described in example 1),
13.5 g of sulphoryl chloride.

This mixture was thereupon irradiated under stirring, with an Osram "Sonne"® 300 Watt lamp, for 30 minutes, during which the internal (inside) temperature rose to 45°C showing a development of gas.

After cooling down to room temperature, the solvent was evaporated at reduced pressure, thereby obtained a residue (32 grams) consisting of the desired product.

*Elemental analysis:*
Cl, in %, theoretical: 44.60 — found: 42.63
P, in %: theoretical: 9.79 — found 9.80
*IR* ($cm^{-1}$): 1265, 1230, 1170, 1025, 970.

Example 4

Preparation of the diethyl ester of 3,3,3-trichloro-1-propene-phosphonic acid: (Reaction 7)

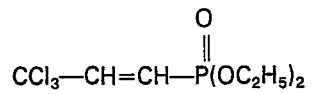

Into a beaker of 300 ml holding capacity, fitted with a magnetical stirrer and a reflux condenser, closed by a calcium chloride valve, there were introduced 16 g of diethyl ester of 2,3,3,3-tetrachloropropane-phosphonic acid (prepared as described in example 3):
50 ml of n-hexane;
6 ml of diethylamine.

This mixture was then subjected to stirring at room temperature for 3 hours whereafter it was allowed to rest overnight. After filtering the chlorohydrate that had formed, the filtrate was washed with 10 ml of HCl 2N, then with water until obtaining a neutral pH. After anhydration with anhydrous $Na_2SO_4$ and subsequent evaporation of the solvent at reduced pressure, there was obtained a residue (12.4 g) consisting of the desired product (b.p. = 84°—85°C at 0.11 mbar.)
*IR* ($cm^{-1}$): 1635; 1260; 1210; 1165; 1020; 970;
*NMR*δ, ppm, ($CDCl_3$, TMS):

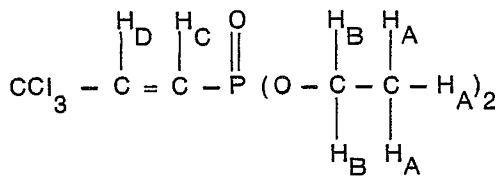

1.40 (t, 6H, $CH_3$)
4.18 (dq, 4H, $CH_2$)
6.32 (dd, 1H, $H_C$)
7.10 (dd, 1H, $H_D$)
$^JH_A—H_B$ =7 Hz
$^JH_B—P$ = 9 Hz
$^JH_C—H_D$ = 16 Hz
$^JH_C—P$ = 14.5 Hz
$^JH_D—P$ = 20 Hz
(t = triplet; dq = doublet of quartet; dd = doublet of doublet; J = coupling constant).

Example 5

Preparation of the diethyl ester of 1,3,3-trichloro-2-propene-phosphonic acid (Reaction 8):

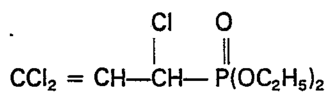

Into a 100 ml beaker, fitted with a magnetical stirrer and with a reflux condenser closed by a calcium chloride valve, there were introduced:
2.8 g of diethyl ester of 3,3,3-trichloro-1-propene-phosphonic acid (obtained as described in example 4), 1 g of $CuCl_2 . 2H_2O$.

This mixture was thereupon stirred for 1 hour and 15 minutes at 110°C. After cooling down to room temperature, it was additioned with 100 ml of ethyl ether and then filtered.

The filtrate was then washed with water until achieving the full disappearance of the green colour, after which it was anhydrified on anhydrous $Na_2SO_4$. The solvent was then evaporated under reduced pressure, thereby obtaining 25 g of the desired product (b.p. = 95°—96°C/0.11 mbar).

*Elemental analysis*:
C, in %: theoretical = 29.87 — found = 29.21
H, in %: theoretical = 4.3 — found = 4.01
P, in %: theoretical = 11.0 – found = 10.91
Cl, in %: theoretical = 37.78 — found = 36.90

*IR* ($cm^{-1}$): 1615, 1270, 1025, 980, 920

*NMR*δ, ppm ($CDCl_3$, TMS)

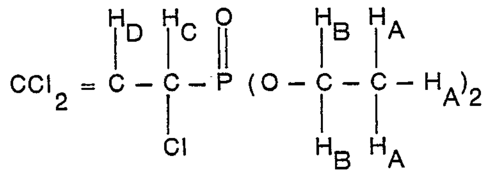

1.39 (t, 6H, $CH_3$)
3.85—4.50 (m, 4H, $CH_2$)
4.7 (dd, 1H, $H_C$)
6.08 (dd, 1H, $H_D$)
$^JH_A$—$H_B$ = 5.5 Hz
$^JH_C$—$H_D$ = 10 Hz
$^JH_C$—P = 13 Hz
$^JH_D$—P = 6 Hz
(t = triplet, m = multiplet, dd = doublet of doublet, J = coupling constant).

Example 6

Preparation of diethyl ester of 3-chloro-2-butene-phosphonic acid. (Reaction 1)

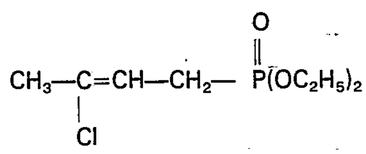

Into a 100 ml flask, fitted with a magnetical stirrer and a reflux condenser, there were introduced:
33.2 g (35 ml, 0.2 mols) of triethylphosphite
25 g (22 ml; 0.2 mols) of 1,3-dichloro-2-butene

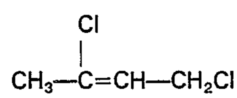

The reaction mixture was thereupon subjected to stirring for 4 hours at 150°C, whereafter it was allowed to cool down to room temperature. Then it was distilled at reduced pressure, gathering the fraction with a b.p. of 145°—146°C at 20 mbar (37 g) and consisting of the desired product.

*NMR*δ, ppm ($CDCl_3$, TMS)

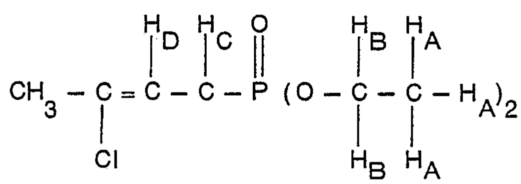

1.1—1.5 (t, 6H, $H_A$)
2.0—2.2 (d, 3H, $CH_3$—C=)
2.4—3.0 (dd, 2H, $H_C$)
4.0—4.3 (dq, 4H, $H_B$)
5.4—5.7 (m, 1h, $H_D$)
$^JH_A$—$H_B$ = 8 Hz
$^JH_B$—P = 8 Hz
$^JH_D$—$H_C$ = 8 Hz
$^JH_C$—P = 20 Hz
(d = doublet; t = triplet; dd = doublet of doublet; dq = doublet of quadrouplet; m = multiplet; J = coupling constant).

Example 7

Preparation of O-ethyl-O-(2′-chloroethyl)-3,3-dichloro-2-propene-phosphonate. (Reaction 2

Into a 50 ml flask, fitted with a magnetical stirrer, a thermometer, a reflux condenser and connected with a $H_2SO_4$ valve, under a nitogen atmosphere and at room temperature, there loaded:
—14.5 g (0.1 mols) of 1,1,3-trichloro-propene
—13.5 g (0.1 mols) of 2-ethoxy-1,3,2-dioxaphospholane of the formula:

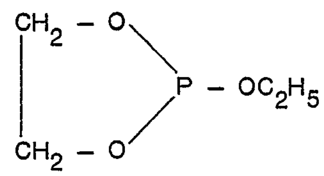

The reaction mixture was then heated up to 140°C and then, in 2 hours and 30 minutes, the temperature was brought up to 165°C. The reaction mixture was thereupon allowed to cool down to room temperature and was then distilled at reduced pressure, gathering the fraction that distilled at between 120° and 130°C at a pressure of 0.27 mbar. The gathered fraction (15.3 g) proved to consist of the desired product showing a purity of 92% (GLC).

*IR* ($cm^{-1}$): 1620 (ν C=C), 1260 (ν P=O), 1025, 970, 920 (ν P—O—C)

*NMR* ($CDCl_3$, TMS)

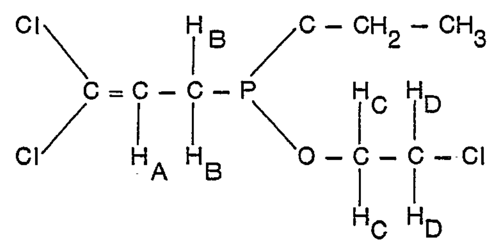

δ (ppm): 1.2—1.5 (t, 3H, $CH_3$)
2.5—3.1 (dd, 2H, $H_B$)
3.6—3.8 (t, 2H, $H_D$)
4.0—4.5 (m, 4H, $CH_2$—$CH_3$ + $H_C$)
5.7—6.15 (m, 1H, $H_A$)
$^{J}CH_3$—$CH_2$ = 7 Hz
$^{J}H_B$—P = 22 Hz
$^{J}H_B$—$H_A$ = 8 Hz
$^{J}H_D$—$H_C$ = 5 Hz
(dd = doublet of doublet; t = triplet; m = multiplet; J = coupling constant).

Example 8

(Preparation of Insecticides)

a) Preparation of ethyl ester of 2,2-dimethyl-3-(4′,4′-dichlorobutadienyl)-cyclopropanecarboxylic acid (Reaction 9):

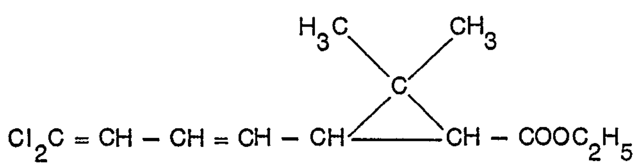

Into a 500 ml flask, anhydrous and maintained under a nitrogen atmosphere, there were introduced 12.5 g of a suspension of about 50% of sodium hydride (0.26 mols) in paraffine oil. This suspension was then washed with anhydrous petroleum ether (2 × 50 ml) and to it were then admixed 20C ml of anhydrous dimethoxytethane.

To this mixture, kept under stirring at 5°—10°C under a nitrogen atmosphere, there was slowly dripped, in a period of 2 hours, a mixture consisting of:

—49.5 g (0.2 mols) of diethyl ester of 3,3-dichloro-2-propene-phosphonic acid (obtained as described in example 1),

—0.24 mols of ethyl ester of the caronic aldehyde (cistrans mixture in a ratio of 58:42).

Once the admixture had been accomplished, the mixture was allowed to spontaneously heat up to room temperature while keeping it under constant stirring for 1 hour.

The reaction mixture was then cooled down to 10°C and to it were then admixed dropwise 5 ml of ethyl alcohol and then 150 ml of a 1% hydrochloric acid. The mixture was then extracted with ethyl ether (2 × 100 ml). The reunited organic phases were then washed with water until reaching a neutral pH, anhydrified on anhydrous sodium sulphate and the solvent eliminated under reduced pressure.

Thereby were obtained 76.2 g of a raw product (dark oil) which was distilled under reduced pressure

thereby gathering the fraction with b.p. of 105°—109°C at 0.067 mbar (19 grams) consisting of the desired product (mixture of geometrical isomers) with a purity degree of 93% (GLC).

*Elemental analysis*:
C, in %: theoretical = 54.77 — found = 53.77
H, in %: theoretical = 6.13 — found = 6.18
Cl, in %: theoretical = 26.95 — found = 25.65

The IR and NMR spectroscopic data were consistent with the assigned structure.

b) Preparation of the chloride of 3-(4′,4′-dichlorobutadienyl)-2,2-dimethyl-cyclopropanecarboxylic acid (trans).
0.002 mols of the product, obtained as described in part a), were treated with 2.6 ml of a 10% solution of KOH in methanol. It was then reflux heated for 4 hours by a water bath. Thereafter it was cooled down and then poured into 50 ml of water. It was then extracted with toluene (2 times, each time with 20 ml of solvent).

The organic phase was then washed with 20 ml of a 10% NaOH. The aqueous phases were then acidified with HCl at 5% concentration and then extracted with toluene (3 times, each time with 25 ml of solvent). The organic phase was thereupon washed with water until attaining a neutral pH, whereafter it was anhydrified on sodium sulphate and then evaporated, thereby obtaining 0.4 g of an oil consisting of the desired product.
I.R. ($cm^{-1}$): 3500—2500—1700—1680; 1430; 1240; 1105; 890; 730; 690.

The raw product, obtained by means of the above described reaction, was dissolved in 3.5 ml of hexane. Then, at 0°—5°C, there were dripped in 3.5 m.mols of thionyl chloride. This mixture was then subjected to reflux and stirring for 4 hours. Thereafter the hexane was decanted from the tars and evaporated at 40°C under a pressure of 20 mbar, thereby obtaining about 0.2 g of chloride of 2,2-dimethyl-3-(4′,4′-dichlorobutadienyl)-cyclopropanecarboxylic acid.
I.R., ν (C=O): 1765 $cm^{-1}$
ν($Cl_2C$=CH): 1580 $cm^{-1}$
(CH=CH): 1615 $cm^{-1}$

c) Preparation of the α-cyano-3-phenoxybenzyl ester of the (±)-trans-3-(4′,4′-dichlorobutadienyl)-2,2-dimethyl-cyclopropanecarboxylic acid.

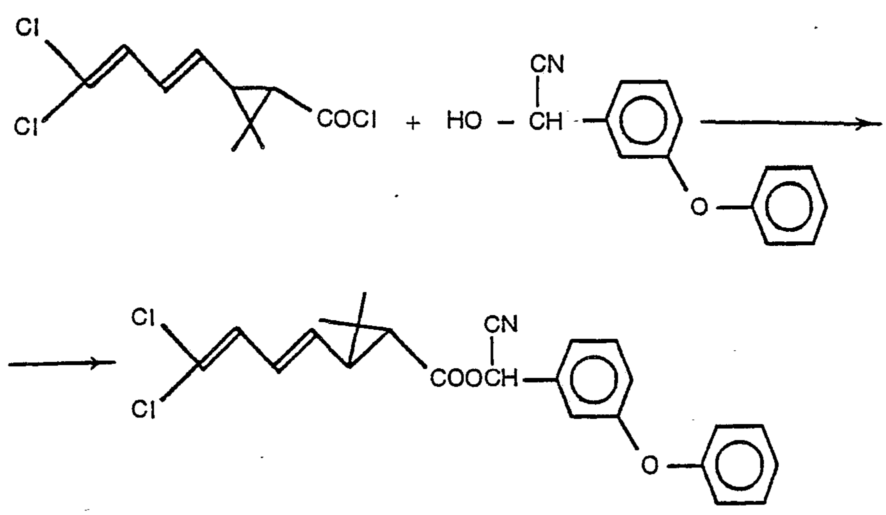

0.2 g of the acid chloride, obtained according to part b), where diluted with 1 ml of anhydrous ethylether. This mixture was cooled down to 0°C and then there was dripped into it a mixture of the cyanhidrine of phenoxybenzaldehyde (180 mg = about 0.8 m.mols) in 0.5 ml of anhydrous ether. This mixture was subjected to stirring at room temperature for 1 hour, whereafter it was cooled down to 0°C and into it was dripped a mixture consisting of 0.75 mols of pyridine and 0.5 ml of ether. Thereupon the temperature was allowed to slowly rise. The mixture was then maintained under stirring at room temperature overnight.

The mixture was then diluted with 5 ml of benzene, washed with water up to a neutral pH (2 × 5 ml), anhydrified, filtered and evaporated whereby there was obtained an oily residue of about 400 mg. Thereupon was rapidly carried out a chromatography with 20 g of silica, eluting with hexaneethyl acetate (9:1). There were obtained 200 mg of a pure product.

GLC titre = 90% (glass column, length = 1.2 m, outside diameter =6 mm, inside diameter = 4 mm;

packed with Chromosorb®W.H.P. 0.15—0.18 mm (80—100 mesh), covered with silicon oil HCC—W982; 3.8% by weight, temperature 280°C, isothermic).

NMR ($CDCl_3$) ν, ppm (TMS): 1.1—1.4 ($CH_3$); 1.6—1.8 (m, γCH—CO) 2.0—2.5 (m, γCH—C=C); 5.0—5.8 (m, *CH*=CH—CH=$CCl_2$); 6.2—6.5 (m, CH—CN and CH=*CH*=$CCl_2$); 6.9—7.6 (m, CH=$CCl_2$ and aromatic protons).

**Claims**

1. Compounds of the general formula:

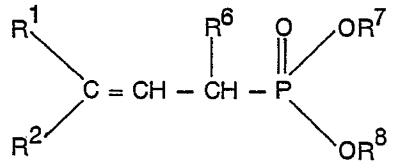

(I)

wherein:

$R^1$ = F, Cl, Br, $CH_3$, $CF_3$;
$R^2$ = F, Cl, Br, $CF_3$;
$R^6$ = H, F, Cl, Br;
$R^7$ and $R^8$, equal to or different from each other, = alkyl or haloalkyl with $C_1$—$C_4$, or $R^7$ and $R^8$ together constitute a

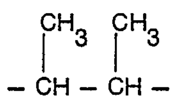 group or a 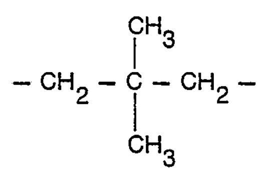 group,

provided that when $R^1$ = $CH_3$, $R^2$ will be different from Cl.

2. Process for the preparation of the compounds according to claim 1, having the formula (I) wherein $R^6$ = Cl, Br, characterized in that a compound of formula:

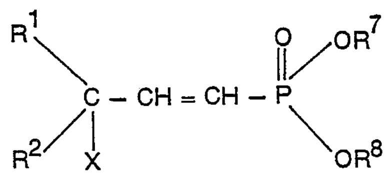

(IV)

(wherein X = Cl, Br and $R^1$, $R^2$, $R^7$ and $R^8$ have the meanings indicated in claim 1) is isomerized at a temperature comprised between 20° and 150°C, in the presence of a cupric halide.

3. Process according to claim 2, characterized in that the isomerization of compound IV is achieved by stirring of a mixture of the above indicated compound with the cupric halide.

**Patentansprüche:**

1. Verbindungen der allgemeinen Formel

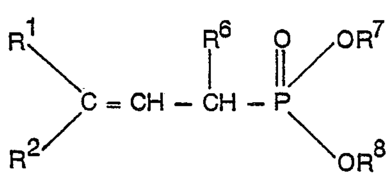

(I)

worin

$R^1$ = F, Cl, Br, $CH_3$, $CF_3$;
$R^2$ = F, Cl, Br, $CF_3$;
$R^6$ = H, F, Cl, Br;
$R^7$ und $R^8$, die gleich oder voneinander verschieden sind = Alkyl oder Halogenalkyl mit $C_1$—$C_4$, oder $R^7$ und $R^8$ bilden gemeinsam eine

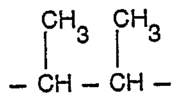 oder eine 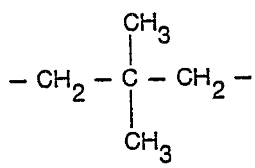 Gruppe

mit der Bedingung, daß, wenn $R^1=CH_3$ ist, $R^2$ eine andere Bedeutung als Cl hat.

2. Verfahren zur Herstellung der Verbindung nach Anspruch 1 mit der Formel (I), in welcher $R^6$ = Cl, Br ist, dadurch gekennzeichnet, daß man eine Verbindung der Formel

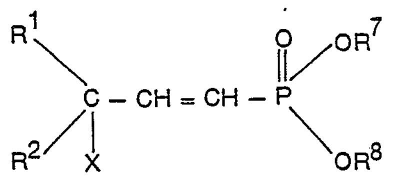

(IV)

(worin X = Cl, Br, und $R^1$, $R^2$, $R^7$ und $R^8$ die in Anspruch 1 angegebene Bedeutung haben) bei einer Temperatur von 20 bis 150°C in Anwesenheit eines Kupfer-II-halogenids isomerisiert.

3. Verfahren nach Anspruch 2, dadurch gekennzeichnet, daß die Isomerisation von Verbindung IV durch Rühren einer Mischung der oben angegebenen Verbindung mit Kupfer-II-halogenid erfolgt.

**Revendications**

1. Composés de formule générale:

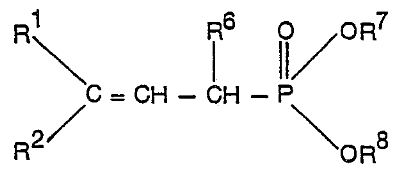

(I)

dans laquelle:

$R^1$ = F, Cl, Br, $CH_3$, $CF_3$;

$R^2$ = F, Cl, Br, $CF_3$;

$R^6$ = H, F, Cl, Br;

$R^7$ et $R^8$ étant identiques ou différents l'un de l'autre, représentent un alkyle ou un haloalkyle en $C_1$ à $C_4$, ou $R^7$ et $R^8$ forment ensemble un groupe

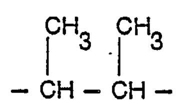 ou un groupe 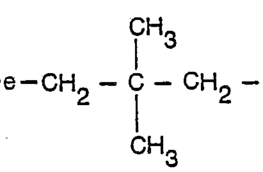

pourvu que, lorsque $R^1$ = $CH_3$, $R^2$ soit différent de Cl.

2. Procédé pour la préparation des composés selon la revendication 1, de formule (I) dans laquelle $R^6$ = Cl, Br, caractérisé ce que un composé de formule:

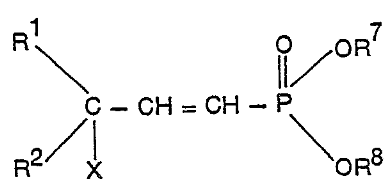

(IV)

dans laquelle:

X = Cl, Br; et

$R^1$, $R^2$, $R^7$ et $R^8$ ont les significations indiquées dans la revendication 1, est isomérisé à une température comprise entre 20° et 150°C, en présence d'un halogénure cuprique.

3. Procédé selon la revendication 2, caractérisé en ce que l'on obtient l'isomérisation du composé IV par agitation d'un mélange du composé indiqué ci-dessus en présence d'halogénure cuprique.